(19) Europäisches Patentamt
European Patent Office
Office européen des brevets

(11) Veröffentlichungsnummer : **0 510 658 B1**

(12) **EUROPÄISCHE PATENTSCHRIFT**

(45) Veröffentlichungstag der Patentschrift : **27.07.94 Patentblatt 94/30**

(51) Int. Cl.$^5$ : **C12N 15/15**, C12N 15/61, C12N 1/38, C12P 1/00

(21) Anmeldenummer : **92106978.7**

(22) Anmeldetag : **23.04.92**

# (54) Verbesserung der Ausbeute bei der Sekretion von disulfidverbrückten Proteinen.

(30) Priorität : **26.04.91 DE 4113750**

(43) Veröffentlichungstag der Anmeldung : **28.10.92 Patentblatt 92/44**

(45) Bekanntmachung des Hinweises auf die Patenterteilung : **27.07.94 Patentblatt 94/30**

(84) Benannte Vertragsstaaten : **AT BE CH DE DK ES FR GB GR IT LI LU NL PT SE**

(56) Entgegenhaltungen :
**EP-A- 0 293 793**
**EP-A- 0 315 782**
**EP-A- 0 393 725**
**J. BIOL. CHEM. Bd. 265, Nr. 28, 1990, Seiten 16760 - 16766 G. BOWDEN ET AL. 'Folding and aggregation of b-lactamase in the periplasmic space of Escherichia coli'**

(73) Patentinhaber : **BOEHRINGER MANNHEIM GMBH**
**D-68298 Mannheim (DE)**

(72) Erfinder : **Glockshuber, Rudolf, Dr.**
**Am Ellbach 3**
**W-8411 Adlmannstein (DE)**
Erfinder : **Wunderlich, Martina**
**Nothaftstrasse 5**
**W-8400 Regensburg (DE)**
Erfinder : **Skerra, Arne, Dr.**
**Cheruskerweg 6**
**W-6200 Wiesbaden (DE)**
Erfinder : **Rudolph, Rainer, Prof.Dr.**
**Färbergasse 17**
**W-8120 Weilheim (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**EP 0 510 658 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Steigerung der Ausbildung der natürlichen Proteinkonformation bei der Sekretion von disulfidverbrückten Proteinen durch einen prokaryontischen Wirtsorganismus, der eine rekombinante DNA enthält, die für das sekretierte Protein kodiert.

Die Proteinsynthese oder Translation in prokaryontischen Mikroorganismen findet an den Ribosomen im Cytoplasma statt. Bei einer Expression von rekombinanter DNA in bakteriellen Wirtsorganismen ist es oft wünschenswert, daß das resultierende rekombinante Genprodukt bzw. Protein aus dem Cytoplasma durch die innere bakterielle Membran in den periplasmatischen Raum zwischen innerer und äußerer Membran sekretiert wird. Vom Periplasma können die sekretierten Proteine dann beispielsweise durch einen osmotischen Schock in das Nährmedium freigesetzt werden. Ein Nachteil dieses Verfahrens ist, daß bei Sekretion von disulfidverbrückten Proteinen in der Regel keine korrekte Ausbildung der nativen bzw. natürlichen Konformation erfolgt, d.h. es entstehen Polypeptide mit einer falschen oder unvollständigen Ausbildung der Disulfidbrücken, die biologisch inaktiv sind.

Thiolreagenzien werden bei Verfahren zur in vitro Renaturierung von unlöslichen Proteinen eingesetzt, die bei Expression von rekombinanter DNA in prokaryontischen Zellen cytoplasmatisch als Inclusion Bodies entstehen. Von den Thiolreagenzien ist bekannt, daß sie in Anwesenheit von Luftsauerstoff rasch zu Disulfiden oxidiert werden.

Eine Aufgabe der vorliegenden Erfindung war die Steigerung der Ausbildung der natürlichen Proteinkonformation bei der Sekretion von disulfidverbrückten Proteinen.

Insbesondere könnte eine Unterstützung der Ausbildung der nativen Proteinkonformation die teilweise recht aufwendige in vitro-Renaturierung der falsch disulfidverbrückten Proteine ersetzen.

Gelöst wird die erfindungsgemäße Aufgabe durch ein Verfahren zur Steigerung der Ausbildung der natürlichen Proteinkonformation bei der Sekretion von disulfidverbrückten Proteinen durch einen prokaryontischen Wirtsorganismus, der eine rekombinante DNA enthält, die für das sekretierte Protein kodiert, worin man den Wirtsorganismus in einem geeigneten Nährmedium unter geeigneten Bedingungen für die Expression der rekombinanten DNA züchtet, wobei das Verfahren dadurch gekennzeichnet ist, daß man ein Nährmedium mit einem Gehalt von 0,1 bis 20 mmol/l eines oder mehrerer Thiolreagenzien verwendet.

Durch das erfindungsgemäße Verfahren gelingt es überraschenderweise, durch Zusatz von Thiolreagenzien im Fermentationsmedium die Ausbeute an korrekt disulfidverbrückten Proteinen zu steigern. Das erfindungsgemäße Verfahren kann bei allen Proteinen angewendet werden, die eine oder mehrere Disulfidbrücken enthalten. Insbesondere bei Bedarf an geringen Proteinmengen, z.B. bei der Produktion von Wachstumsfaktoren im mg-Maßstab (Nerve Growth Factor, Interleukine oder ähnliche Verbindungen) kann durch Anwendung des erfindungsgemäßen Verfahrens auf eine in vitro-Renaturierung verzichtet werden.

Für das erfindungsgemäße Verfahren hat sich ein Nährmedium mit einem Gehalt von 0,1 bis 20 mmol/l eines oder mehrerer Thiolreagenzien als geeignet erwiesen. Verwendet man ein Nährmedium mit einem Gehalt von weniger als 0,1 mmol/l an Thiolreagenzien, so wird keine nennenswerte Steigerung der Ausbildung der natürlichen Proteinkonformation gefunden. Die Obergrenze der Thiolkonzentration liegt etwa bei 20 mmol/l und manifestiert sich sowohl in einer deutlichen Abnahme der Ausbeuten an sekretiertem, funktionalem Protein als auch in einem starken Rückgang des Zellenwachstums. Vorzugsweise verwendet man das Nährmedium mit einem Gehalt von 1 bis 15 mmol/l und besonders bevorzugt mit einem Gehalt von 3 bis 12 mmol/l eines oder mehrerer Thiolreagenzien. Bei Verwendung von Glutathion als Thiolreagenz haben sich etwa 5 mmol/l als optimale Konzentration erwiesen.

Der in der vorliegenden Beschreibung verwendete Begriff "Thiolreagenz" bedeutet entweder ein reduzierendes Thiolreagenz mit SH-Gruppen oder ein Gemisch von reduzierenden Thiolreagenzien mit SH-Gruppen und oxidierenden Thiolreagenzien mit Disulfidgruppen.

Als reduzierende Thiolreagenzien sind insbesondere solche geeignet, die eine einzige SH-Gruppe pro Molekül aufweisen. Besonders bevorzugte Substanzen sind reduziertes Glutathion (GSH), Cystein, N-Acetylcystein, Cysteamin, β-Mercaptoethanol und ähnliche Verbindungen. Am meisten bevorzugt sind N-Acetylcysein und reduziertes Glutathion (GSH). Die Thiolreagenzien können sowohl einzeln als auch in Gemischen verwendet werden.

Obwohl die alleinige Verwendung reduzierender Thiolreagenzien im allgemeinen bevorzugt ist, bringt auch die Verwendung eines Gemisches von reduzierenden und oxidierenden Thiolreagenzien ein verbesserte Ausbeute an korrekt disulfidverbrückten Proteinen. Bei Verwendung eines derartigen Gemisches von reduzierenden und oxidierenden Thiolreagenzien beträgt das molare Verhältnis von reduzierenden zu oxidierenden Thiolreagenzien vorzugsweise 2:1 bis 20:1, besonders bevorzugt 5:1 bis 10:1. Ein Gemisch eines reduzierten und eines oxidierten Thiolreagenzes sind beispielsweise reduziertes Glutathion (GSH) und Glutathiondisulfid (GSSG).

Ein Beispiel für das erfindungsgemäße Verfahren ist die heterologe Expression des bifunktionalen α-Amylase/Trypsin-Inhibitors aus Eleusine coracana Gaertneri (RBI) in E.coli. Der Inhibitor wurde von Shivaraj und Pattabiraman charakterisiert (Shivaraj B. & Pattabiraman, T.N., Indian J.Biochem.Biophys. 17 (1980), 181-193; Shivaraj B. & Pattabiraman, T.N.,Biochem.J. 193 (1981), 29-36). Die Aminosäuresequenz des Inhibitors wurde von Campos und Richardson aufgeklärt (Campos, F.A.P. & Richardson, M., FEBS Letters 152 (1983), 300-304). Dieses Protein besteht aus 122 Aminosäuren, enthält 5 intramolekulare Disulfidbrücken und gehört einer neuen α-Amylase/Trypsin-Inhibitorklasse an (Halford et al., Biochim.Biophys.Acta 950 (1988),435-440), wobei es deren einziger bifunktionaler Vertreter ist. An dieser Stelle soll jedoch angemerkt werden, daß sich das erfindungsgemäße Verfahren auch auf die Gewinnung anderer disulfidverbrückter rekombinanter Proteine (z.B. Antikörperfragmente) in prokaryontischen Wirtsorganismen übertragen läßt, die sekretiert werden.

Zur Gewinnung des sekretorischen RBI-Proteins in funktionaler Form in E.coli wurde ein synthetisches RBI-Gen mit gentechnologischen Mitteln an die Signalsequenz des Outer Membrane Proteins A (OmpA) von E.coli fusioniert und die Fusion auf einem rekombinanten Plasmid unter Kontrolle eines lac-Promotors in E.coli exprimiert. Auf diese Weise wird die Polypeptidkette des rekombinanten Proteins ins Periplasma der prokaryontischen Wirtszelle transportiert, wo sie sich nach Abspaltung der Signalsequenz aufgrund der oxidierenden Eigenschaften dieses Zellkompartiments unter Ausbildung der intramolekularen Disulfidbrücken zum nativen Protein falten kann. Bei dieser Faltung können jedoch nur geringe Mengen des funktionalen Proteins erhalten werden. Bei der erfindungsgemäßen Anwesenheit von Thiolreagenzien im Nährmedium gelingt es jedoch, die Ausbeute an funktionalem Protein erheblich (um den Faktor 5) zu steigern.

Der Wirtsorganismus für das erfindungsgemäße Verfahren ist ein prokaryontischer Wirtsorganismus. Vorzugsweise führt man die Expression des rekombinanten Proteins in einem gram-negativen prokaryontischen Wirtsorganismus, besonders bevorzugt in E.coli durch.

Bei dem erfindungsgemäßen Verfahren ist es im allgemeinen günstig, daß die für das sekretierte Protein kodierende rekombinante DNA in operativer Verknüpfung mit einem DNA-Fragment steht, das für ein Signalpeptid zum Durchdringen von inneren bakteriellen Membranen kodiert. Der Begriff "operative Verknüpfung" im Sinne der vorliegenden Erfindung bedeutet, daß eine translationale Fusion zwischen dem heterologen Protein und dem Signalpeptid besteht. Dabei bildet das Signalpeptid üblicherweise den N-terminalen Anteil der translationalen Fusion. Die Art des Signalpeptids selbst ist für die vorliegende Erfindung nicht kritisch, abgesehen davon, daß es die Sekretion des rekombinanten Proteins ermöglichen soll. Eine große Anzahl derartiger Signalpeptide sind dem Fachmann auf dem Gebiet der Molekularbiologie bekannt. Eine Reihe von Signalpeptiden sind z.B. bei Winnacker (Gene und Klone, Eine Einführung in die Gentechnologie, Verlag Chemie Weinheim (1985), S. 256) aufgezählt. Wird das erfindungsgemäße Verfahren in E.coli als Wirtsorganismus durchgeführt, so hat sich das Signalpeptid aus dem E.coli OmpA-Protein als besonders geeignet erwiesen.

Für die Expression in einem prokaryontischen Wirtsorganismus muß die rekombinante DNA, welche für das sekretierte Protein kodiert, unter Kontrolle eines vom Transkriptionssystem des Wirts erkannten Expressionssignals bzw. Promotors stehen. Derartige, in prokaryontischen Wirtszellen aktive Expressionssignale sind dem Fachmann auf dem Gebiet der Molekularbiologie bekannt. Vorzugsweise wird für das erfindungsgemäße Verfahren ein induzierbares Expressionssignal verwendet. Beispiele für einen induzierbaren E.coli-Promotor sind der lac-Promotor, der durch Isopropyl-β-D-galactosid (IPTG) induzierbar ist, sowie synthetische Derivate des lac-Promotors (z.B. der tac- oder trc-Promotor).

Die rekombinante DNA, die für das disulfidverbrückte sekretierte Protein kodiert, wird im allgemeinen durch Transformation in die prokaryontische Wirtszelle eingeführt. Techniken zur Transformation verschiedener prokaryontischer Wirtsorganismen sind dem Fachmann bekannt und brauchen daher nicht im einzelnen genannt werden. Die in der transformierten Wirtszelle vorliegende rekombinante DNA befindet sich dabei üblicherweise auf einem rekombinanten Vektor, der in der Wirtszelle entweder extrachromosomal (z.B. Plasmid) oder im Genom der Wirtszelle integriert (z.B. Bakteriophage λ) vorliegen kann. Vorzugsweise wird als Vektor ein Plasmid verwendet. Dabei ist es für das erfindungsgemäße Verfahren nicht kritisch, welches spezielle Plasmid dabei als Expressionsvektor verwendet wird. Es kommt nur darauf an, daß die für das gewünschte Protein kodierende rekombinante DNA von der Wirtszelle in ausreichendem Maße transkribiert und translatiert werden kann. Das Translationsprodukt der rekombinanten DNA muß allerdings in einer Form vorliegen, die eine Sekretion durch die innere bakterielle Membran in das Periplasma erlaubt.

Wie oben ausgeführt, wird üblicherweise ein rekombinantes Protein, das mit einer Signalsequenz fusioniert ist, ins Periplasma einer prokaryontischen Wirtszelle transportiert. Dies stellt auch eine bevorzugte Ausführungsform der vorliegenden Erfindung dar. Bei Verwendung bestimmter Wirtsstämme erfolgt jedoch nicht nur eine Sekretion ins Periplasma, sondern auch eine massive Proteinsekretion in das Nährmedium. Die EP-A 0 338 410 offenbart E.coli-Stämme, die zu einer derartigen massiven Proteinsekretion in das Nährmedium in der Lage sind sowie ein Verfahren zur Herstellung derartiger Stämme. Als Ausgangsstämme für die Herstellung dieser Sekretormutanten werden vorzugsweise die in der EP-A 0 338 410 genannten E.coli-Stämme DS410 (DSM 4513) bzw. BW7261 (DSM 5231) verwendet.

Als Nährmedium für das erfindungsgemäße Verfahren wird vorzugsweise ein Vollmedium verwendet, insbesondere ein Medium, das Substanzen oder Substanzgemische aus Zellextrakten enthält. Ein Beispiel für ein solches Medium ist das LB-Medium, das Substanzgemische aus enzymatisch verdauten Zellextrakten (Bacto-Trypton und Yeast-Extract) enthält.

Der pH-Wert des Nährmediums liegt vorzugsweise in einem Bereich zwischen pH 5 und pH 9. Besonders bevorzugt liegt der pH-Wert des Nährmediums zwischen 6 und 8. Verwendet man LB-Medium als Nährmedium, so hat es sich als günstig erwiesen, einen pH-Wert von 7,4 bei der Herstellung des Mediums einzustellen. Während des Zellwachstums sinkt dann der pH-Wert des Mediums ab und liegt erfahrungsgemäß bei stationären Übernachtkulturen (Zeitpunkt der Zellernte) bei etwa 6,8.

Das erfindungsgemäße Verfahren wird vorzugsweise in einer Schüttelkultur in einem Vollmedium durchgeführt. Es ist jedoch auch möglich, die Wirtsorganismen in einem stark belüfteten Fermenter oder/und in Gegenwart eines Minimalmediums zu züchten.

Eine weitere Ausführungsform des erfindungsgemäßen Verfahrens besteht darin, daß man dem Nährmedium weiterhin ein oder mehrere Mono- oder/und Oligosaccharide zusetzt, die vom verwendeten prokaryontischen Wirtsorganismus nicht metabolisiert werden können. Aus einer Arbeit von Bowden und Georgiou (J.Biol. Chem. 265 (1990), 16760-16766) ist nämlich bekannt, daß der Zusatz von nicht metabolisierbaren Zuckern die Ausbildung der nativen Proteinkonformation fördern kann. Die zusätzliche Verwendung dieser nicht metabolisierbaren Zucker im Rahmen des erfindungsgemäßen Verfahrens zeigt einen weiteren unterstützenden Effekt bei der Ausbildung der nativen Proteinkonformation von sekretierten Proteinen. Beispiele für nicht-metabolisierbare Zucker sind etwa Sorbose (ein Monosaccharid), Saccharose (ein Disaccharid) und Raffinose (ein Trisaccharid). Die Konzentration der nicht-metabolisierbaren Mono- oder/und Oligosaccharide im Nährmedium liegt für das erfindungsgemäße Verfahren günstigerweise zwischen 0,1 mol/l und 1 mol/l, vorzugsweise zwischen 0,3 mol/l und 0,7 mol/l.

Eine weitere besonders bevorzugte Ausführungsform des erfindungsgemäßen Verfahrens besteht darin, daß man in einem Wirtsorganismus zusammen mit der Expression der für das zu sekretierende, disulfidverbrückte Protein kodierenden rekombinanten DNA eine Überexpression eines Proteindisulfidisomerasegens durchführt. Vorzugsweise verwendet man ein Proteindisulfidisomerasegen aus gram-negativen Bakterienspezies, besonders bevorzugt ein Proteindisulfidisomerasegen aus E.coli. Die DNA-Sequenz eines derartigen Gens ist in SEQ ID NO: 3 gezeigt.

Überraschenderweise wurde festgestellt, daß eine Coexpression und Cosekretion eines wirtseigenen Proteindisulfidisomerasegens in Kombination mit der Zugabe von Thiolreagenzien in das Nährmedium eine Ausbeutesteigerung an funktionalem, sekretiertem Protein bewirkt, die über die alleinige Wirkung des Zusatzes von Thiolreagenzien noch hinausgeht. Dagegen hat die alleinige Coexpression von Proteindisulfidisomerase (PDI) ohne Zugabe von Thiolreagenzien keine Ausbeuteerhöhung zur Folge.

Der Begriff "Überexpression" gemäß vorliegender Erfindung bedeutet eine Steigerung der Proteindisulfidisomerase-Expression im Vergleich zu einem Wildtyp des jeweils verwendeten prokaryontischen Wirtsorganismus. Eine derartige Überexpression läßt sich beispielsweise dadurch erreichen, daß sich das Proteindisulfidisomerasegen unter Kontrolle eines starken, vorzugsweise induzierbaren Expressionssignals (z.B. eines lac-Promotors oder Derivaten davon) befindet. Vorzugsweise erfolgt die Überexpression des PDI-Gens in "operativer Verknüpfung" mit einem Signalpeptid zum Durchdringen innerer bakterieller Membranen. Besonders bevorzugt wird zu diesem Zweck die natürliche PDI-Signalsequenz verwendet.

In einer Ausführungsform des erfindungsgemäßen Verfahrens kann beispielsweise die für das zu sekretierende Protein kodierende rekombinante DNA und das Protendisulfidisomerasegen auf einem einzigen Expressionsvektor in der Wirtszelle vorliegen. Dieser Expressionsvektor ist vorzugsweise ein extrachromosomaler Vektor, d.h. ein Plasmid, er kann jedoch auch integriert im Chromosom der Wirtszelle vorliegen (z.B. Lambda-Phage). Bei Verwendung eines einzigen Expressionsvektors ist es bevorzugt, daß sich die rekombinante DNA und das Proteindisulfidisomerasegen unter Kontrolle eines einzigen Expressionssignals, d.h. in Form eines dicistronischen Operons befinden.

Andererseits ist es jedoch möglich, daß sich die für das zu sekretierende Protein kodierende rekombinante DNA und das Proteindisulfidisomerasegen auf zwei miteinander kompatiblen extrachromosomalen Expressionsvektoren jeweils unter Kontrolle eines eigenen Expressionssignals (Promotors) befinden.

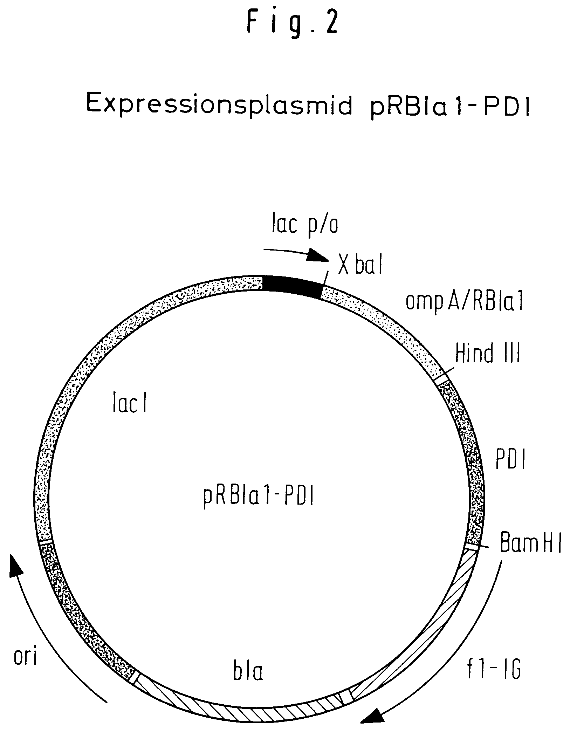

Die vorliegende Erfindung soll weiterhin durch die folgenden Beispiele in Verbindung mit den Abbildungen 1 und 2 sowie den SEQ ID NO: 1 - 5 verdeutlicht werden.

| | |
|---|---|
| Abb. 1 | zeigt einen Immunblot zum Nachweis von löslichem, prozessiertem RBI nach Zugabe unterschiedlicher Mengen von Glutathion ins Nährmedium. |
| Abb. 2 | zeigt eine schematische Darstellung des Expressionsplasmids pRBIa1-PDI. |
| SEQ ID NO:1 | zeigt die DNA-Sequenz des zur Coexpression von RBI und der Proteindisulfidisomerase |

(PDI) aus E.coli W3110 verwendeten Plasmids pRBIa1-PDI.

SEQ ID NO:2 zeigt die Sequenz des OmpA/RBI Gens. Die doppelsträngige DNA wird aus 14 synthetischen Oligonukleotiden zusammengesetzt und durch die Restriktionsschnittstellen XbaI und HindIII begrenzt. Der im Sequenzprotokoll gezeigte DNA-Strang ist aus 7 Oligonukleotiden zusammengesetzt, die folgenden Abschnitten entsprechen:

| | | | | |
|---|---|---|---|---|
| 1 : | Basen | 2 | - | 62 |
| 2 : | Basen | 63 | - | 126 |
| 3 : | Basen | 127 | - | 195 |
| 4 : | Basen | 196 | - | 258 |
| 5 : | Basen | 259 | - | 322 |
| 6 : | Basen | 323 | - | 396 |
| 7 : | Basen | 397 | - | 455 |

Der Gegenstrang ist ebenfalls aus 7 Oligonukleotiden gebildet, die zu folgenden Abschnitten des gezeigten DNA-Stranges komplementär sind:

| | | | | |
|---|---|---|---|---|
| 8 : | Basen | 6 | - | 68 |
| 9 : | Basen | 69 | - | 132 |
| 10: | Basen | 133 | - | 201 |
| 11: | Basen | 202 | - | 264 |
| 12: | Basen | 265 | - | 328 |
| 13: | Basen | 329 | - | 402 |
| 14: | Basen | 403 | - | 459. |

SEQ ID NO: 3 zeigt die Sequenz des Proteindisulfidisomerasegens mit der natürlichen Signalsequenz und ribosomaler Bindungsstelle.

SEQ ID NO: 4 zeigt den N-terminalen Primer zur Amplifizierung des PDI-Gens.

SEQ ID NO: 5 zeigt den C-terminalen Primer zur Amplifizierung des PDI-Gens.

Beispiel 1

Gensynthese

Verbemerkung:

Molekulargenetische Techniken beruhten auf Maniatis et al. (Molecular Cloning. A Laboratory Manual (1982), Cold Spring Harbor Laboratory, New York), Oligonukleotide wurden nach dem Phosphoramiditverfahren (Sinha et al., Nucl.Acids Res. 12 (1984), 4539-4557; Kaplan, B.E., Trends Biotechnol.3 (1985), 253-256) auf einem automatischen Synthesegerät Typ 380A der Firma Applied Biosystems GmbH hergestellt.

Das synthetische Gen, das für die Fusion zwischen der OmpA-Signalsequenz und RBI kodiert, wurde aus 14 synthetischen Oligonukleotiden zusammengesetzt (SEQ ID NO: 2). Die Oligonukleotide wurden durch Polyacrylamid/Harnstoff-Gelelektrophorese gereinigt und mit Ausnahme der beiden überstehenden Oligonukleotide am 5′- und am 3′-Ende des Gens jeweils an ihren 5′-Enden phosphoryliert. Danach wurden alle Oligonukleotide in äquimolaren Verhältnissen vereinigt und in einem einzigen Ansatz hybridisiert und ligiert. Das Gen wird durch die Restriktionsstellen XbaI und HindIII begrenzt. Die Aminosäuren der OmpA-Signalsequenz sind mit negativem Vorzeichen gekennzeichnet.

Beispiel 2

Konstruktion des Expressionsplasmids pRBIa 1

Das nach Beispiel 1 erhaltene synthetische Gen wurde über die Restriktionsstellen XbaI und HindIII in das Expressionsplasmid pASK40 (Skerra et al., BIO/TECHNOLOGY 9 (1991), 273-278) kloniert. Die Sequenz des synthetischen Gens wurde durch Didesoxy-Sequenzierung überprüft. Das resultierende Plasmid wurde pRBIa1 bezeichnet.

Beispiel 3

Funktionale Expression von RBI im Periplasma von E.coli

Eine stationäre Übernachtkultur von E.coli JM83 (Yannish-Perron et al., Gene 33 (1985), 103-119), die mit pRBIa1 transformiert worden war, wurde in Verhältnis 1:100 in 2,5 l LB-Medium (1 l LB-Medium enthält 10 g Bacto-Trypton (Difco Factories, Detroit, Michigan, USA), 5 g Yeast-Extrakt (Difco Factories) und 5 g NaCl, pH 7,4) mit Ampicillin (100 µg/ml) verdünnt und bei 26°C geschüttelt, bis eine $OD_{550}$ von 1,0 erreicht war. Danach wurde die Kultur in 9 identische 250 ml-Portionen unterteilt, jede Kultur mit IPTG (Isopropyl-β-D-galactosid; Endkonzentration 1 mmol/l) induziert, jedoch mit unterschiedlichen Mengen Glutathion (GSH) versetzt. Die Zellen wurden über Nacht bei 26°C weitergeschüttelt und durch Zentrifugation geerntet (Sorvall SS34, 4°C, 5000 Upm, 15 Minuten). Danach wurden die Zellen bei 4°C in 100 mmol/l Tris/HCl pH 7,5, 20 mmol/l Na-EDTA aufgenommen, so daß Zelldichten von jeweils 200 ($OD_{550}$) erhalten wurden. Die Zellen wurden darauf in einer French Pressure-Zellpresse (Aminco) bei 18000 PSI lysiert. Die Lysate wurden zentrifugiert (Sorvall SS34, 4°C, 15000 Upm, 30 Minuten) und die löslichen Überstände auf ihren Gehalt an löslichem, funktionalem RBI untersucht.

Jeweils 5 µl der erhaltenen löslichen Überstände wurden auf einem 15 %igen Polyacrylamid/SDS-Gel aufgetrennt (Fling & Gregerson, Anal.Biochem. 155 (1986) 83-88). Die aufgetrennten Proteine wurden durch Elektroelution auf ein Nitrocellulosemembran übertragen und die RBI-Banden mit Hilfe eines Anti-RBI-Kaninchenantikörpers immunspezifisch gefärbt. Das Immunblotting wurde nach Blake et al., Anal.Biochem. 136 (1984), 175-179 durchgeführt.

Abb. 1 zeigt eine Analyse der Expressionsausbeuten an löslichem RBI durch Immunblotting. Spur 1 stellt einen Molekulargewichtsstandard dar. In Spur 2 wurden 0,8 µg RBI, gereinigt aus Eleusine coracana Gaertneri aufgetragen. Auf den Spuren 3 bis 6 wurden äquivalente Mengen der Zellextrakte mit unterschiedlichen Zugabemengen an Glutathion aufgetragen. In Spur 3 erfolgte keine GSH-Zugabe zum Kulturmedium. In Spuren 4, 5 und 6 wurden dem Kulturmedium 1 mmol/l, 5 mmol/l bzw. 10 mmol/l GSH zugesetzt.

Als Antikörper wurde ein polyklonales Anti-RBI-Antiserum verwendet, das nach Standardmethoden (siehe z.B. Sambrook et al., Molecular Cloning. A Laboratory Manual (1989), Cold Spring Harbor Laboratory Press, Kapitel 18) durch zweimalige Immunisierung eines Neuseelandkaninchens mit gereinigtem RBI aus Eleusine coracana Gaertneri hergestellt wurde.

Aus der Abbildung geht hervor, daß der Zusatz von GSH die Intensität der immunspezifisch angefärbten Bande verstärkt. Dies ist insbesondere bei Konzentrationen von 5 mmol/l und 10 mmol/l GSH ersichtlich.

Durch Bestimmung der inhibitorischen Aktivität gegenüber Trypsin aus Rinderpankreas wurde die RBI-Menge in den löslichen Anteilen der erhaltenen Zellextrakte quantitativ bestimmt. Es konnte gezeigt werden, daß die Menge an intrazellulärem, funktionalem Inhibitor bis zum 5-fachen gegenüber der Expression ohne die beschriebenen Mediumzusätze gesteigert werden kann (Tabelle 1).

Ferner ist aus Tabelle 1 ersichtlich, daß auch durch Zusatz eines Gemisches von reduziertem Glutathion (GSH) und Glutathiondisulfid (GSSG) eine Verbesserung der Ausbeute an funktionalem Inhibitor erhalten wird.

Bei allen durchgeführten Tests wurden 5 µg Trypsin aus Rinderpankreas in 1 ml 100 mmol/l NaCl, 50 mmol/l Tris/HCl pH 8,0, 10 mmol/l $CaCl_2$, 0,005 % (v/v) Triton X-100 mit der zu bestimmenden Menge an RBI 30 Minuten bei 25°C inkubiert. Nach Zugabe von 20 µl 10 mmol/l N-α-Benzoyl-L-arginin-4-nitranilid (chromogenes Testsubstrat) wurde die Restaktivität des Trypsins durch Registrierung der zeitlichen Zunahme der Absorption bei 405 nm bestimmt. Die Inhibitorkonzentration im Test wurde aus der Differenz zwischen der Aktivität des freien Enzyms (ohne Zugabe des Inhibitors) und der jeweils gemessenen Restaktivität ermittelt.

Tabelle 1 zeigt die Expressionssteigerung von funktionalem RBI durch Zusatz von GSH bzw. eines Gemisches aus GSH und GSSG.

Tabelle 1

| | mg / l · OD* | relative Zunahme | mg/l* | relative Zunahme |
|---|---|---|---|---|
| ohne GSH | 0,07 | 1,0 | 0,36 | 1,0 |
| 5 mmol/l GSH | 0,37 | 5,3 | 1,65 | 4,6 |
| 10 mmol/l GSH | 0,34 | 4,9 | 1,38 | 3,8 |
| 5 mmol/l GSH + 1 mol/l GSSG | 0,25 | 3,9 | 1,18 | 2,7 |

* Die Ausbeuten an intrazellulärem, funktionalem RBI sind in mg / l · OD (Liter x optische Dichte), die Volumenausbeuten in mg/l angegeben.

Beispiel 4

Verwendung von N-Acetyl-L-Cystein als weiteres Thiolreagenz

Wie im Beispiel 3 beschrieben, werden Kulturen von E.coli JM83, die mit dem Plasmid pRBIa1 transformiert worden waren, bei 26°C im LB-Medium mit Ampicillin (100 µg/ml) vermehrt und bei einer $OD_{550}$ = 1 mit IPTG (Endkonzentration: 1 mmol/l) induziert. Gleichzeitig erfolgte die Zugabe von N-Acetyl-L-Cystein in fester form (Endkonzentration: 5 und 10 mmol/l). Die Kulturen wurden dann über Nacht weitergeschüttelt und, wie in Beispiel 3 beschrieben, durch Trypsin-Inhibitionstests auf ihren Gehalt an funktionalem RBI untersucht. Die Ergebnisse sind in Tabelle 2 zusammengefaßt und zeigen, daß der Konzentrationsbereich, in dem das N-Acetyl-L-Cystein wirkt, dem des reduzierenden Glutathions sehr ähnlich ist.

Tabelle 2

Expression von funktionalem RBI durch Zusatz von N-Acetyl-Cystein

| Zusatz im Medium | mg/l·OD* | relative Zunahme | mg/l* | relative Zunahme |
|---|---|---|---|---|
| ohne Zusatz | 0,07 | 1,0 | 0,36 | 1,0 |
| 5 mmol/l N-Acetyl-Cystein | 0,31 | 4,4 | 1,27 | 3,5 |
| 10 mmol/l N-Acetyl-Cystein | 0,25 | 3,6 | 0,83 | 2,3 |
| 5 mmol/l GSH | 0,37 | 5,3 | 1,65 | 4,6 |

* Die Ausbeuten an intrazellulärem, funktionalem RBI sind in mg/l·OD, die Volumenausbeuten in mg/l angegeben. Die Bestimmung der RBI-Aktivität erfolgte durch Trypsin-Inhibitionstests im löslichen Teil des Gesamtzellextrakts (vgl. Beispiel 3).

Beispiel 5

Coexpression und Cosekretion von periplasmatischer Proteindisulfidisomerase (PDI) und gleichzeitige Zugabe von Thiolreagenzien ins Kulturmedium

Zur Coexpression der wirtseigenen, periplasmatischen Proteindisulfidisomerase (PDI) wurde zunächst das PDI-Gen aus dem Genom des Ecoli K12 Wildtypstamms W3110 (Bachmann, B.J. (1972), Bacteriol.Rev. 36, 525-557) durch Polymerase-Kettenreaktion (PCR) amplifiziert. Dabei diente die bekannte Basensequenz des Gens (Bardwell, J.C.A., McGovern, K.& Beckwith, J., "Identification of a Protein Required for Disulfide Bond Formation in vivo", Cell, 67, (1991), 581-589) als Ausgangspunkt für die Planung der entsprechenden Oligonukleotidprimer.

Zur Amplifizierung des PDI-Gens wurden folgende Oligonukleotide als Primer verwendet:

N-terminaler Primer: (SEQ ID NO: 4)

5' TTGCAATTAACAAAGCTTGAATTCTCGGAGAGAGTAGATCATGAAAAAGAT 3'

C-terminaler Primer: (SEQ ID NO: 5)

5' GGGCTTTATGTAAAGCTTGGATCCTTATTTTTTCTCGGACAGATATTTC 3'

Das amplifizierte DNA-Fragment, das das komplette Gen der PDI inklusive Signalsequenz und ribosomaler Bindungsstelle enthält, wurde isoliert und mit den Restriktionsenzymen HindIII und BamHI geschnitten. Das Plasmid pRBIa1 wurde mit denselben Enzymen verdaut, das Vektorfragment isoliert und mit dem PDI-Gen ligiert. Das resultierende Plasmid pRBIa1-PDI enthält die Gene für OmpA/RBI und PDI als dicistronisches Operon unter der Kontrolle des lac-Promotors (Abb. 2). Die vollständige Nukleotidsequenz des Plasmids ist in SEQ ID NO: 1 dokumentiert.

Zur Analyse des Effekts der Coexpression und Cosekretion von PDI wurde E.coli JM83 mit dem Plasmid pRBI-PDI transformiert.

Die Anzucht und die Bestimmung der gebildeten intrazellulären RBI-Mengen erfolgte wie in den Beispielen 3 und 4 beschrieben. Die Analyse ergab, daß die Coexpression und Cosekretion von PDI nur bei gleichzeitiger Zugabe von reduziertem Glutathion zu einer Ausbeutesteigerung führt, die erheblich über der bei alleiniger Zugabe von Glutathion beobachteten Ausbeutesteigerung liegt, während die alleinige Expression von PDI überhaupt keinen Effekt auf die Menge an funktionalem, periplasmatischem RBI hat (Tabelle 3).

Tabelle 3

Expressionssteigerung von funktionalem RBI durch Koexpression von PDI aus E.coli

| Zusatz im Medium | mg/l·OD* | relative Zunahme | mg/l* | relative Zunahme |
|---|---|---|---|---|
| ohne Zusatz | 0,08 | 1,0 | 0,38 | 1,0 |
| 5 mmol/l GSH | 0,37 | 4,6 | 1,65 | 4,3 |
| PDI | 0,08 | 1,0 | 0,39 | 1,0 |
| PDI + 1 mM GSH | 0,42 | 6,0 | 1,71 | 4,4 |
| PDI + 5 mM GSH | 0,97 | 13,7 | 4,33 | 11,1 |
| PDI + 10 mM GSH | 0,54 | 7,7 | 2,17 | 5,6 |
| PDI + 1 mM GSH + 0,5 mM GSSG | 0,38 | 5,4 | 2,00 | 5,1 |
| PDI + 5 mM GSH + 0,5 mM GSSG | 0,60 | 8,6 | 2,54 | 6,5 |
| PDI + 10 mM GSH + 0,5 mM GSSG | 0,46 | 6,6 | 1,93 | 4,9 |
| PDI + 0,5 mM GSSG | 0,10 | 1,4 | 0,57 | 1,5 |

* Die Ausbeuten an intralzellulärem, funktionalem RBI sind in mg/l·OD, die Volumenausbeuten in mg/l angegeben. Die Bestimmung der RBI-Aktivität erfolgte durch Trypsin-Inhibitionstests im löslichen Teil des Gesamtzellextrakts (vgl. Beispiel 3).

SEQUENZPROTOKOLL

SEQ ID NO:1:

LÄNGE: 4690 Basenpaare
TYP: Nukleinsäure mit korrespondierendem Protein
STRANGFORM: Doppelstrang
TOPOLOGIE: zirkulär

NAME: Signalpeptid OmpA
POSITION: 1328..1391

NAME: reifes Peptid RBI
POSITION: 1392..1756

NAME: Signalpeptid
POSITION: 1789..1845

NAME: reifes Peptid PDI
OSITION: 1846..2412

```
ACCCGACACC ATCGAATGGC GCAAAACCTT TCGCGGTATG GCATGATAGC GCCCGGAAGA     60
GAGTCAATTC AGGGTGGTGA ATGTGAAACC AGTAACGTTA TACGATGTCG CAGAGTATGC    120
CGGTGTCTCT TATCAGACCG TTTCCCGCGT GGTGAACCAG GCCAGCCACG TTTCTGCGAA    180
AACGCGGGAA AAAGTGGAAG CGGCGATGGC GGAGCTGAAT TACATTCCCA ACCGCGTGGC    240
ACAACAACTG GCGGGCAAAC AGTCGTTGCT GATTGGCGTT GCCACCTCCA GTCTGGCCCT    300
GCACGCGCCG TCGCAAATTG TCGCGGCGAT TAAATCTCGC GCCGATCAAC TGGGTGCCAG    360
CGTGGTGGTG TCGATGGTAG AACGAAGCGG CGTCGAAGCC TGTAAAGCGG CGGTGCACAA    420
TCTTCTCGCG CAACGCGTCA GTGGGCTGAT CATTAACTAT CCGCTGGATG ACCAGGATGC    480
CATTGCTGTG GAAGCTGCCT GCACTAATGT TCCGGCGTTA TTTCTTGATG TCTCTGACCA    540
GACACCCATC AACAGTATTA TTTTCTCCCA TGAAGACGGT ACGCGACTGG GCGTGGAGCA    600
TCTGGTCGCA TTGGGTCACC AGCAAATCGC GCTGTTAGCG GGCCCATTAA GTTCTGTCTC    660
GGCGCGTCTG CGTCTGGCTG GCTGGCATAA ATATCTCACT CGCAATCAAA TTCAGCCGAT    720
AGCGGAACGG GAAGGCGACT GGAGTGCCAT GTCCGGTTTT CAACAAACCA TGCAAATGCT    780
GAATGAGGGC ATCGTTCCCA CTGCGATGCT GGTTGCCAAC GATCAGATGG CGCTGGGCGC    840
AATGCGCGCC ATTACCGAGT CCGGGCTGCG CGTTGGTGCG GATATCTCGG TAGTGGGATA    900
CGACGATACC GAAGACAGCT CATGTTATAT CCCGCCGTTA ACCACCATCA AACAGGATTT    960
TCGCCTGCTG GGGCAAACCA GCGTGGACCG CTTGCTGCAA CTCTCTCAGG GCCAGGCGGT   1020
GAAGGGCAAT CAGCTGTTGC CCGTCTCACT GGTGAAAAGA AAAACCACCC TGGCGCCCAA   1080
```

```
TACGCAAACC GCCTCTCCCC GCGCGTTGGC CGATTCATTA ATGCAGCTGG CACGACAGGT    1140

TTCCCGACTG GAAAGCGGGC AGTGAGCGCA ACGCAATTAA TGTGAGTTAG CTCACTCATT    1200

AGGCACCCCA GGCTTTACAC TTTATGCTTC CGGCTCGTAT GTTGTGTGGA ATTGTGAGCG    1260

GATAACAATT TCACACAGGA AACAGCTATG ACCATGATTA CGAATTTCTA GATAACGAGG    1320

GCAAAAA ATG AAA AAG ACA GCT ATC GCG ATT GCA GTG GCA CTG GCC GGC      1369
        Met Lys Lys Thr Ala Ile Ala Ile Ala Val Ala Leu Ala Gly
        -21 -20                 -15                 -10

TTC GCT ACC GTA GCG CAG GCC TCT GTT GGT ACC TCT TGC ATC CCG GGT      1417
Phe Ala Thr Val Ala Gln Ala Ser Val Gly Thr Ser Cys Ile Pro Gly
         -5                  1               5

ATG GCT ATC CCG CAC AAC CCA CTG GAC TCT TGT AGA TGG TAT GTG TCG      1465
Met Ala Ile Pro His Asn Pro Leu Asp Ser Cys Arg Trp Tyr Val Ser
 10                  15                  20                  25

ACC CGC ACC TGC GGG GTT GGC CCT AGA CTG GCT ACT CAA GAA ATG AAA      1513
Thr Arg Thr Cys Gly Val Gly Pro Arg Leu Ala Thr Gln Glu Met Lys
                 30                  35                  40

GCT CGT TGC TGC CGT CAG CTC GAG GCT ATC CCG GCG TAC TGT CGT TGC      1561
Ala Arg Cys Cys Arg Gln Leu Glu Ala Ile Pro Ala Tyr Cys Arg Cys
             45                  50                  55

GAA GCT GTT CGT ATC CTG ATG GAC GGT GTT GTG ACG TCT TCT GGT CAG      1609
Glu Ala Val Arg Ile Leu Met Asp Gly Val Val Thr Ser Ser Gly Gln
         60                  65                  70

CAC GAA GGT CGT CTC CTG CAG GAT CTC CCA GGT TGC CCG CGT CAG GTA      1657
His Glu Gly Arg Leu Leu Gln Asp Leu Pro Gly Cys Pro Arg Gln Val
     75                  80                  85

CAG CGT GCT TTC GCT CCG AAA CTG GTT ACT GAA GTT GAA TGC AAC CTG      1705
Gln Arg Ala Phe Ala Pro Lys Leu Val Thr Glu Val Glu Cys Asn Leu
 90                  95                 100                 105

GCG ACT ATC CAT GGT GGC CCG TTC TGC CTG TCT CTG CTG GGT GCT GGT      1753
Ala Thr Ile His Gly Gly Pro Phe Cys Leu Ser Leu Leu Gly Ala Gly
                110                 115                 120

GAA TGATAAGCTT GAATTCTCGG AGAGAGTAGA TC ATG AAA AAG ATT TGG CTG      1806
Glu                                     Met Lys Lys Ile Trp Leu
                                        -19             -15

GCG CTG GCT GGT TTA GTT TTA GCG TTT AGC GCA TCG GCG GCG CAG TAT      1854
Ala Leu Ala Gly Leu Val Leu Ala Phe Ser Ala Ser Ala Ala Gln Tyr
            -10                 -5                   1

GAA GAT GGT AAA CAG TAC ACT ACC CTG GAA AAA CCG GTA GCT GGC GCG      1902
Glu Asp Gly Lys Gln Tyr Thr Thr Leu Glu Lys Pro Val Ala Gly Ala
     5                  10                  15
```

```
CCG CAA GTG CTG GAG TTT TTC TCT TTC TTC TGC CCG CAC TGC TAT CAG     1950
Pro Gln Val Leu Glu Phe Phe Ser Phe Phe Cys Pro His Cys Tyr Gln
 20                 25                  30                  35

TTT GAA GAA GTT CTG CAT ATT TCT GAT AAT GTG AAG AAA AAA CTG CCG     1998
Phe Glu Glu Val Leu His Ile Ser Asp Asn Val Lys Lys Lys Leu Pro
                40                  45                  50

GAA GGC GTG AAG ATG ACT AAA TAC CAC GTC AAC TTC ATG GGT GGT GAC     2046
Glu Gly Val Lys Met Thr Lys Tyr His Val Asn Phe Met Gly Gly Asp
            55                  60                  65

CTG GGC AAA GAT CTG ACT CAG GCA TGG GCT GTG GCG ATG GCG CTG GGC     2094
Leu Gly Lys Asp Leu Thr Gln Ala Trp Ala Val Ala Met Ala Leu Gly
        70                  75                  80

GTG GAA GAC AAA GTG ACT GTT CCG CTG TTT GAA GGC GTA CAG AAA ACC     2142
Val Glu Asp Lys Val Thr Val Pro Leu Phe Glu Gly Val Gln Lys Thr
    85                  90                  95

-AG ACC ATT CGT TCT GCT TCT GAT ATC CGC GAT GTA TTT ATC AAC GCA     2190
Gln Thr Ile Arg Ser Ala Ser Asp Ile Arg Asp Val Phe Ile Asn Ala
100                 105                 110                 115

GGT ATT AAA GGT GAA GAG TAC GAC GCG GCG TGG AAC AGC TTC GTG GTG     2238
Gly Ile Lys Gly Glu Glu Tyr Asp Ala Ala Trp Asn Ser Phe Val Val
                120                 125                 130

AAA TCT CTG GTC GCT CAG CAG GAA AAA GCT GCA GCT GAC GTG CAA TTG     2286
Lys Ser Leu Val Ala Gln Gln Glu Lys Ala Ala Ala Asp Val Gln Leu
            135                 140                 145

CGT GGC GTT CCG GCG ATG TTT GTT AAC GGT AAA TAT CAG CTG AAT CCG     2334
Arg Gly Val Pro Ala Met Phe Val Asn Gly Lys Tyr Gln Leu Asn Pro
        150                 155                 160

CAG GGT ATG GAT ACC AGC AAT ATG GAT GTT TTT GTT CAG CAG TAT GCT     2382
Gln Gly Met Asp Thr Ser Asn Met Asp Val Phe Val Gln Gln Tyr Ala
    165                 170                 175

GAT ACA GTG AAA TAT CTG TCC GAG AAA AAA TAAGGATCCC CACGCGCCCT       2432
Asp Thr Val Lys Tyr Leu Ser Glu Lys Lys
180                 185

GTAGCGGCGC ATTAAGCGCG GCGGGTGTGG TGGTTACGCG CAGCGTGACC GCTACACTTG   2492

CCAGCGCCCT AGCGCCCGCT CCTTTCGCTT TCTTCCCTTC CTTTCTCGCC ACGTTCGCCG   2552

GCTTTCCCCG TCAAGCTCTA AATCGGGGCA TCCCTTTAGG GTTCCGATTT AGTGCTTTAC   2612

GGCACCTCGA CCCCAAAAAA CTTGATTAGG GTGATGGTTC ACGTAGTGGG CCATCGCCCT   2672

GATAGACGGT TTTTCGCCCT TTGACGTTGG AGTCCACGTT CTTTAATAGT GGACTCTTGT   2732

TCCAAACTGG AACAACACTC AACCCTATCT CGGTCTATTC TTTTGATTTA TAAGGGATTT   2792

TGCCGATTTC GGCCTATTGG TTAAAAAATG AGCTGATTTA ACAAAAATTT AACGCGAATT   2852
```

```
TTAACAAAAT ATTAACGTTT ACAATTTCAG GTGGCACTTT TCGGGGAAAT GTGCGCGGAA    2912
CCCCTATTTG TTTATTTTTC TAAATACATT CAAATATGTA TCCGCTCATG AGACAATAAC    2972
CCTGATAAAT GCTTCAATAA TATTGAAAAA GGAAGAGTAT GAGTATTCAA CATTTCCGTG    3032
TCGCCCTTAT TCCCTTTTTT GCGGCATTTT GCCTTCCTGT TTTTGCTCAC CCAGAAACGC    3092
TGGTGAAAGT AAAAGATGCT GAAGATCAGT TGGGTGCACG AGTGGGTTAC ATCGAACTGG    3152
ATCTCAACAG CGGTAAGATC CTTGAGAGTT TTCGCCCCGA AGAACGTTTT CCAATGATGA    3212
GCACTTTTAA AGTTCTGCTA TGTGGCGCGG TATTATCCCG TATTGACGCC GGGCAAGAGC    3272
AACTCGGTCG CCGCATACAC TATTCTCAGA ATGACTTGGT TGAGTACTCA CCAGTCACAG    3332
AAAAGCATCT TACGGATGGC ATGACAGTAA GAGAATTATG CAGTGCTGCC ATAACCATGA    3392
GTGATAACAC TGCGGCCAAC TTACTTCTGA CAACGATCGG AGGACCGAAG GAGCTAACCG    3452
CTTTTTTGCA CAACATGGGG GATCATGTAA CTCGCCTTGA TCGTTGGGAA CCGGAGCTGA    3512
ATGAAGCCAT ACCAAACGAC GAGCGTGACA CCACGATGCC TGTAGCAATG GCAACAACGT    3572
TGCGCAAACT ATTAACTGGC GAACTACTTA CTCTAGCTTC CCGGCAACAA TTAATAGACT    3632
GGATGGAGGC GGATAAAGTT GCAGGACCAC TTCTGCGCTC GGCCCTTCCG GCTGGCTGGT    3692
TTATTGCTGA TAAATCTGGA GCCGGTGAGC GTGGGTCTCG CGGTATCATT GCAGCACTGG    3752
GGCCAGATGG TAAGCCCTCC CGTATCGTAG TTATCTACAC GACGGGGAGT CAGGCAACTA    3812
TGGATGAACG AAATAGACAG ATCGCTGAGA TAGGTGCCTC ACTGATTAAG CATTGGTAAC    3872
TGTCAGACCA AGTTTACTCA TATATACTTT AGATTGATTT AAAACTTCAT TTTTAATTTA    3932
AAAGGATCTA GGTGAAGATC CTTTTTGATA ATCTCATGAC CAAAATCCCT TAACGTGAGT    3992
TTTCGTTCCA CTGAGCGTCA GACCCCGTAG AAAAGATCAA AGGATCTTCT TGAGATCCTT    4052
TTTTTCTGCG CGTAATCTGC TGCTTGCAAA CAAAAAAACC ACCGCTACCA GCGGTGGTTT    4112
GTTTGCCGGA TCAAGAGCTA CCAACTCTTT TTCCGAAGGT AACTGGCTTC AGCAGAGCGC    4172
AGATACCAAA TACTGTCCTT CTAGTGTAGC CGTAGTTAGG CCACCACTTC AAGAACTCTG    4232
TAGCACCGCC TACATACCTC GCTCTGCTAA TCCTGTTACC AGTGGCTGCT GCCAGTGGCG    4292
ATAAGTCGTG TCTTACCGGG TTGGACTCAA GACGATAGTT ACCGGATAAG GCGCAGCGGT    4352
CGGGCTGAAC GGGGGGTTCG TGCACACAGC CCAGCTTGGA GCGAACGACC TACACCGAAC    4412
TGAGATACCT ACAGCGTGAG CTATGAGAAA GCGCCACGCT TCCCGAAGGG AGAAAGGCGG    4472
ACAGGTATCC GGTAAGCGGC AGGGTCGGAA CAGGAGAGCG CACGAGGGAG CTTCCAGGGG    4532
```

GAAACGCCTG GTATCTTTAT AGTCCTGTCG GGTTTCGCCA CCTCTGACTT GAGCGTCGAT 4592

TTTTGTGATG CTCGTCAGGG GGGCGGAGCC TATGGAAAAA CGCCAGCAAC GCGGCCTTTT 4652

TACGGTTCCT GGCCTTTTGC TGGCCTTTTG CTCACATG 4690

SEQ ID NO:2:

LÄNGE: 460 Basenpaare
TYP: Nukleinsäure mit korrespondierendem Protein
STRANGFORM: Doppelstrang
TOPOLOGIE: linear

NAME: Signalpeptid
POSITION: 22..85

NAME: Signalpeptide
POSITION: 86..451

```
TCTAGATAAC GAGGGCAAAA A ATG AAA AAG ACA GCT ATC GCG ATT GCA GTG      51
                        Met Lys Lys Thr Ala Ile Ala Ile Ala Val
                        -21 -20                 -15

GCA CTG GCC GGC TTC GCT ACC GTA GCG CAG GCC TCT GTT GGT ACC TCT      99
Ala Leu Ala Gly Phe Ala Thr Val Ala Gln Ala Ser Val Gly Thr Ser
    -10                 -5                   1               5

TGC ATC CCG GGT ATG GCT ATC CCG CAC AAC CCA CTG GAC TCT TGT AGA     147
Cys Ile Pro Gly Met Ala Ile Pro His Asn Pro Leu Asp Ser Cys Arg
                10                  15                  20

TGG TAT GTG TCG ACC CGC ACC TGC GGG GTT GGC CCT AGA CTG GCT ACT     195
Trp Tyr Val Ser Thr Arg Thr Cys Gly Val Gly Pro Arg Leu Ala Thr
            25                  30                  35

CAA GAA ATG AAA GCT CGT TGC TGC CGT CAG CTC GAG GCT ATC CCG GCG     243
Gln Glu Met Lys Ala Arg Cys Cys Arg Gln Leu Glu Ala Ile Pro Ala
        40                  45                  50

TAC TGT CGT TGC GAA GCT GTT CGT ATC CTG ATG GAC GGT GTT GTG ACG     291
Tyr Cys Arg Cys Glu Ala Val Arg Ile Leu Met Asp Gly Val Val Thr
    55                  60                  65

TCT TCT GGT CAG CAC GAA GGT CGT CTC CTG CAG GAT CTC CCA GGT TGC     339
Ser Ser Gly Gln His Glu Gly Arg Leu Leu Gln Asp Leu Pro Gly Cys
70                  75                  80                  85

CCG CGT CAG GTA CAG CGT GCT TTC GCT CCG AAA CTG GTT ACT GAA GTT     387
Pro Arg Gln Val Gln Arg Ala Phe Ala Pro Lys Leu Val Thr Glu Val
                90                  95                  100

GAA TGC AAC CTG GCG ACT ATC CAT GGT GGC CCG TTC TGC CTG TCT CTG     435
Glu Cys Asn Leu Ala Thr Ile His Gly Gly Pro Phe Cys Leu Ser Leu
            105                 110                 115

CTG GGT GCT GGT GAA T GATAAGCTT                                     460
Leu Gly Ala Gly Glu
        120
```

SEQ ID NO:3:

LÄNGE: 639 Basenpaare
TYP: Nukleinsäure mit korrespondierendem Protein
STRANGFORM: Doppelstrang
TOPOLOGIE: linear

NAME: Signalpeptid
POSITION: 16..72

NAME: Signalpeptid
POSITION: 73..639

```
CGGAGAGAGT AGATC ATG AAA AAG ATT TGG CTG GCG CTG GCT GGT TTA GTT     51
                 Met Lys Lys Ile Trp Leu Ala Leu Ala Gly Leu Val
                 -19             -15                 -10

TTA GCG TTT AGC GCA TCG GCG GCG CAG TAT GAA GAT GGT AAA CAG TAC      99
Leu Ala Phe Ser Ala Ser Ala Ala Gln Tyr Glu Asp Gly Lys Gln Tyr
        -5                  1               5

ACT ACC CTG GAA AAA CCG GTA GCT GGC GCG CCG CAA GTG CTG GAG TTT     147
Thr Thr Leu Glu Lys Pro Val Ala Gly Ala Pro Gln Val Leu Glu Phe
 10                  15                  20                  25

TTC TCT TTC TTC TGC CCG CAC TGC TAT CAG TTT GAA GAA GTT CTG CAT     195
Phe Ser Phe Phe Cys Pro His Cys Tyr Gln Phe Glu Glu Val Leu His
                 30                  35                  40

ATT TCT GAT AAT GTG AAG AAA AAA CTG CCG GAA GGC GTG AAG ATG ACT     243
Ile Ser Asp Asn Val Lys Lys Lys Leu Pro Glu Gly Val Lys Met Thr
            45                  50                  55

AAA TAC CAC GTC AAC TTC ATG GGT GGT GAC CTG GGC AAA GAT CTG ACT     291
Lys Tyr His Val Asn Phe Met Gly Gly Asp Leu Gly Lys Asp Leu Thr
        60                  65                  70

CAG GCA TGG GCT GTG GCG ATG GCG CTG GGC GTG GAA GAC AAA GTG ACT     339
Gln Ala Trp Ala Val Ala Met Ala Leu Gly Val Glu Asp Lys Val Thr
    75                  80                  85

GTT CCG CTG TTT GAA GGC GTA CAG AAA ACC CAG ACC ATT CGT TCT GCT     387
Val Pro Leu Phe Glu Gly Val Gln Lys Thr Gln Thr Ile Arg Ser Ala
 90                  95                 100                 105

TCT GAT ATC CGC GAT GTA TTT ATC AAC GCA GGT ATT AAA GGT GAA GAG     435
Ser Asp Ile Arg Asp Val Phe Ile Asn Ala Gly Ile Lys Gly Glu Glu
                110                 115                 120

TAC GAC GCG GCG TGG AAC AGC TTC GTG GTG AAA TCT CTG GTC GCT CAG     483
Tyr Asp Ala Ala Trp Asn Ser Phe Val Val Lys Ser Leu Val Ala Gln
            125                 130                 135
```

```
CAG GAA AAA GCT GCA GCT GAC GTG CAA TTG CGT GGC GTT CCG GCG ATG          531
Gln Glu Lys Ala Ala Ala Asp Val Gln Leu Arg Gly Val Pro Ala Met
        140                 145                 150

TTT GTT AAC GGT AAA TAT CAG CTG AAT CCG CAG GGT ATG GAT ACC AGC          579
Phe Val Asn Gly Lys Tyr Gln Leu Asn Pro Gln Gly Met Asp Thr Ser
    155                 160                 165

AAT ATG GAT GTT TTT GTT CAG CAG TAT GCT GAT ACA GTG AAA TAT CTG          627
Asn Met Asp Val Phe Val Gln Gln Tyr Ala Asp Thr Val Lys Tyr Leu
170                 175                 180                 185

TCC GAG AAA AAA                                                          639
Ser Glu Lys Lys
```

SEQ ID NO.4:

LÄNGE: 51 Basen
TYP: Nukleinsäure
STRANGFORM: Einzelstrang
TOPOLOGIE: linear

```
TTGCAATTAA CAAAGCTTGA ATTCTCGGAG AGAGTAGATC ATGAAAAAGA T                 51
```

SEQ ID NO.5:

LÄNGE: 49 Basen
TYP: Nukleinsäure
STRANGFORM: Einzelstrang
TOPOLOGIE: linear

```
GGGCTTTATG TAAAGCTTGG ATCCTTATTT TTTCTCGGAC AGATATTTC                    49
```

## Patentansprüche

1. Verfahren zur Steigerung der Ausbildung der natürlichen Proteinkonformation bei der Sekretion von disulfidverbrückten Proteinen durch einen prokaryontischen Wirtsorganismus, der eine rekombinante DNA enthält, die für das sekretierte Protein kodiert, worin man den Wirtsorganismus in einem geeigneten Nährmedium unter geeigneten Bedingungen für die Expression der rekombinanten DNA züchtet,
**dadurch gekennzeichnet,**
daß man ein Nährmedium mit einem Gehalt von 0,1 bis 20 mmol/l eines oder mehrerer Thiolreagenzien verwendet.

2. Verfahren nach Anspruch 1,
**dadurch gekennzeichnet,**
daß man ein Nährmedium mit einem Gehalt von 1 bis 15 mmol/l eines oder mehrerer Thiolreagenzien verwendet.

3. Verfahren nach Anspruch 2,
**dadurch gekennzeichnet,**
daß man ein Nährmedium mit einem Gehalt von 3 bis 12 mmol/l eines oder mehrerer Thiolreagenzien verwendet.

4. Verfahren nach einem der Ansprüche 1 bis 3,
**dadurch gekennzeichnet,**
daß man reduzierende Thiolreagenzien mit SH-Gruppen verwendet.

5. Verfahren nach einem der Ansprüche 1 bis 4,
**dadurch gekennzeichnet,**
daß man reduzierende Thiolreagenzien mit einer SH-Gruppe pro Molekül verwendet.

6. Verfahren nach Anspruch 5,
**dadurch gekennzeichnet,**
daß man als reduzierende Thiolreagenzien Glutathion (GSH), Cystein, N-Acetylcystein, Cysteamin, β-Mercaptoethanol oder Gemische davon verwendet.

7. Verfahren nach einem der Ansprüche 1 bis 3,
**dadurch gekennzeichnet,**
daß man ein Gemisch von reduzierenden Thiolreagenzien mit SH-Gruppen und oxidierenden Thiolreagenzien mit Disulfidgruppen verwendet.

8. Verfahren nach Anspruch 7,
**dadurch gekennzeichnet,**
daß das molare Verhältnis von reduzierenden zu oxidierenden Thiolreagenzien 2:1 bis 20:1 beträgt.

9. Verfahren nach einem der Ansprüche 7 bis 8,
**dadurch gekennzeichnet,**
daß man ein Gemisch aus reduziertem Glutathion (GSH) und Glutathiondisulfid (GSSG) verwendet.

10. Verfahren nach Anspruch 9,
**dadurch gekennzeichnet,**
daß die für das sekretierte Protein kodierende rekombinante DNA in operativer Verknüpfung mit einem DNA-Fragment steht, das für ein Signalpeptid zum Durchdringen von inneren bakteriellen Membranen kodiert.

11. Verfahren nach Anspruch 10,
**dadurch gekennzeichnet,**
daß das Signalpeptid aus dem E.coli OmpA Protein stammt.

12. Verfahren nach einem der Ansprüche 1 bis 11,
**dadurch gekennzeichnet,**
daß sich die für das sekretierte Protein kodierende rekombinante DNA unter Kontrolle eines induzierbaren Expressionssignals befindet.

13. Verfahren nach einem der vorhergehenden Ansprüche,
**dadurch gekennzeichnet,**
daß man dem Nährmedium weiterhin ein oder mehrere Mono- oder/und Oligosaccharide zusetzt, die vom Wirtsorganismus nicht metabolisiert werden können.

14. Verfahren nach Anspruch 13,
**dadurch gekennzeichnet,**
daß man ein oder mehrere Mono- oder/und Oligosaccharide ausgewählt aus der Gruppe, bestehend aus Sorbose, Saccharose, Raffinose oder Gemischen davon, verwendet.

15. Verfahren nach einem der vorhergehenden Ansprüche,
**dadurch gekennzeichnet,**
daß man in einem Wirtsorganismus zusammen mit der Expression der für das zu sekretierende, disulfidverbrückte Protein kodierenden rekombinanten DNA eine Überexpression eines Proteindisulfidisomerasegens durchführt.

## Claims

1. Process for increasing the formation of the natural protein conformation when disulfide-bonded proteins are secreted by a prokaryotic host organism that contains a recombinant DNA coding for the secreted protein in which the host organism is cultured in a suitable culture medium under suitable conditions for the expression of the recombinant DNA,
**wherein**
a culture medium containing 0.1 to 20 mmol/l of one or several thiol reagents is used.

2. Process as claimed in claim 1,
**wherein**
a culture medium containing 1 to 15 mmol/l of one or several thiol reagents is used.

3. Process as claimed in claim 2,
**wherein**
a culture medium containing 3 to 12 mmol/l of one or several thiol reagents is used.

4. Process as claimed in one of the claims 1 to 3,
**wherein**
reducing thiol reagents with SH groups are used.

5. Process as claimed in one of the claims 1 to 4,
**wherein**
reducing thiol reagents with one SH group per molecule are used.

6. Process as claimed in claim 5,
**wherein**
glutathione (GSH), cysteine, N-acetylcysteine, cysteamine, β-mercaptoethanol or mixtures thereof are used as the reducing thiol reagents.

7. Process as claimed in one of the claims 1 to 3,
**wherein**
a mixture of reducing thiol reagents with SH groups and oxidizing thiol reagents with disulfide groups is used.

8. Process as claimed in claim 7,
**wherein**
the molar ratio of reducing to oxidizing thiol reagents is 2:1 to 20:1.

9. Process as claimed in one of the claims 7 to 8,
**wherein**
a mixture of reduced glutathione (GSH) and glutathione disulfide (GSSG) is used.

10. Process as claimed in claim 9,
**wherein**
the recombinant DNA coding for the secreted protein is operatively linked with a DNA fragment that codes for a signal peptide for penetrating inner bacterial membranes.

11. Process as claimed in claim 10,
**wherein**
the signal peptide is derived from the E. coli OmpA protein.

12. Process as claimed in one of the claims 1 to 11,
**wherein**
the recombinant DNA coding for the secreted protein is under the control of an inducible expression signal.

13. Process as claimed in one of the previous claims,

**wherein**
one or several monosaccharides or/and oligosaccharides which cannot be metabolized by the host organism are additionally added to the culture medium.

14. Process as claimed in claim 13,
**wherein**
one or several monosaccharides or/and oligosaccharides selected from the group comprising sorbose, sucrose, raffinose or mixtures thereof are used.

15. Process as claimed in one of the previous claims,
**wherein**
an overexpression of a protein disulfide isomerase gene is carried out in a host organism together with the expression of the recombinant DNA which codes for the secreted disulfide-bonded protein.

## Revendications

1. Procédé pour augmenter la formation de la conformation naturelle des protéines lors de la sécrétion de protéines à ponts disulfure par un organisme hôte procaryotique qui contient un ADN recombinant qui code la protéine sécrétée, dans lequel on cultive l'organisme hôte dans un milieu nutritif approprié dans des conditions appropriées pour l'expression de l'ADN recombinant, caractérisé en ce que l'on utilise un milieu nutritif ayant une teneur de 0,1 à 20 mmol/l d'un ou plusieurs réactifs thioliques.

2. Procédé selon la revendication 1, caractérisé en ce que l'on utilise un milieu nutritif ayant une teneur de 1 à 15 mmol/l d'un ou plusieurs réactifs thioliques.

3. Procédé selon la revendication 2, caractérisé en ce que l'on utilise un milieu nutritif ayant une teneur de 3 à 12 mmol/l d'un ou plusieurs réactifs thioliques.

4. Procédé selon l'une des revendications 1 à 3, caractérisé en ce que l'on utilise des réactifs thioliques réducteurs à groupes SH.

5. Procédé selon l'une des revendications 1 à 4, caractérisé en ce que l'on utilise des réactifs thioliques réducteurs ayant un groupe SH par molécule.

6. Procédé selon la revendication 5, caractérisé en ce que l'on utilise comme réactifs thioliques réducteurs le glutathion (GSH), la cystéine, la N-acétylcystéine, la cystéamine, le β-mercaptoéthanol ou des mélanges de ceux-ci.

7. Procédé selon l'une des revendications 1 à 3, caractérisé en ce que l'on utilise un mélange de réactifs thioliques réducteurs à groupes SH et de réactifs thioliques oxydants à groupes disulfure.

8. Procédé selon la revendication 7, caractérisé en ce que le rapport molaire des réactifs thioliques réducteurs aux réactifs thioliques oxydants est de 2:1 à 20:1.

9. Procédé selon l'une des revendications 7 à 8, caractérisé en ce que l'on utilise un mélange de glutathion réduit (GSH) et de disulfure de glutathion (GSSG).

10. Procédé selon la revendication 9, caractérisé en ce que l'ADN recombinant codant la protéine sécrétée est lié de manière active à un fragment d'ADN qui code un peptide signal pour la traversée des membranes bactériennes internes.

11. Procédé selon la revendication 10, caractérisé en ce que le peptide signal provient de la protéine OmpA de E. coli.

12. Procédé selon l'une des revendications 1 à 11, caractérisé en ce que l'ADN recombinant codant la protéine sécrétée se trouve sous le contrôle d'un signal d'expression inductible.

13. Procédé selon l'une des revendications précédentes, caractérisé en ce que l'on ajoute en outre au milieu nutritif un ou plusieurs mono- et/ou oligosaccharides qui ne peuvent pas être métabolisés par l'organisme hôte.

**14.** Procédé selon la revendication 13, caractérisé en ce que l'on utilise un ou plusieurs mono- et/ou oligosaccharides choisis dans le groupe consistant en le sorbose, le saccharose, le raffinose ou des mélanges de ceux-ci.

**15.** Procédé selon l'une des revendications précédentes, caractérisé en ce que l'on réalise une surexpression d'un gène de protéine disulfure isomérase dans un organisme hôte en même temps que l'expression de l'ADN recombinant codant la protéine à ponts disulfure qui doit être sécrétée.

Fig. 1

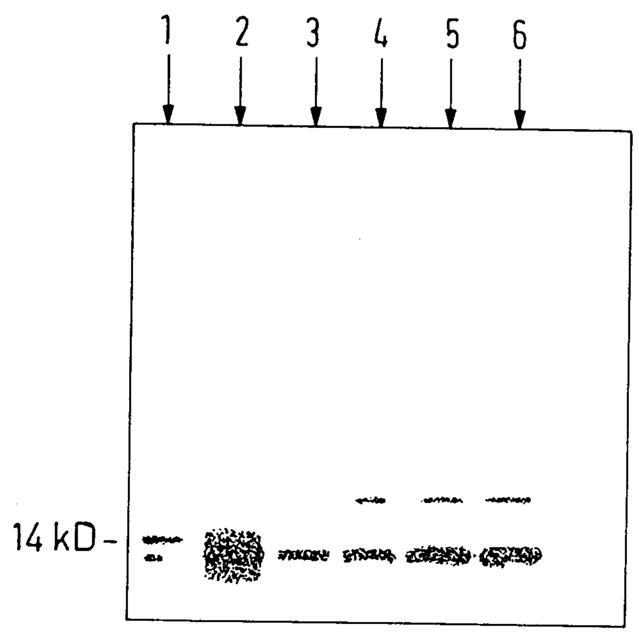

Fig. 2

Expressionsplasmid pRBla1-PDI